# FASCICULE DE BREVET EUROPEEN

(11) **EP 2 735 235 B1**
(45) Date de publication et mention de la délivrance du brevet: **24.04.2019**
(21) Numéro de dépôt: 13290291.7
(22) Date de dépôt: 22.11.2013
(51) Int. Cl.: A23K 20/189, A23K 50/30, A23K 50/75, A23K 50/80, C12N 9/52

(54) **Utilisation d'un complexe enzymatique dans l'alimentation des animaux d'élevage**
Verwendung eines Enzymkomplexes bei der Ernährung von Zuchttieren
Use of an enzyme complex in the feed of farm animals

(30) Priorité: 26.11.2012 FR 1203171
(43) Date de publication de la demande: 28.05.2014
(73) Titulaire: Hooreman, Dominique, 30127 Bellegarde (FR); Hooreman, Anne-Sophie, 62126 Wimille (FR); Hooreman, Caroline, 75017 Paris (FR); Hooreman, Marie, 75017 Paris (FR); Hooreman, Jean-Noël, 75017 Paris (FR); Hooreman, Hervé, 95160 Montmorency (FR)
(72) Inventeur: Hooreman, Dominique, 30127 Bellegarde (FR)
(74) Mandataire: Célanie, Christian

(56) Documents cités:
- WO-A1-87/07905
- WO-A1-97/37681
- FR-A1- 2 034 559
- GB-A- 1 133 579
- MORIHARA K ET AL: "Multiple proteolytic enzymes of Streptomyces fradiae production, isolation, and preliminary characterization", BBA ENZYMOLOGY, ELSEVIER, AMSTERDAM, NL, vol. 139, no. 2, 11 juillet 1967 (1967-07-11), pages 382-397, XP023510232, ISSN: 0005-2744, DOI: 10.1016/0005-2744(67)90042-3 [extrait le 1967-07-11]
- J. C. BLUM ET AL: "INFLUENCE DE LA < FRADIASE >, UN ADDITIF ALIMENTAIRE A ACTIVITÉ PROTÉOLYTIQUE, SUR L'UTILISATION DE LA FARINE DE VIANDE ET LES PERFORMANCES DE LA POULE PONDEUSE", ANNALES DE ZOOTECHNIE, vol. 22, no. 4, 1 janvier 1973 (1973-01-01), pages 493-499, XP055073615, ISSN: 0003-424X, DOI: 10.1051/animres:19730409

## Description

Le secteur technique de la présente invention appartient au domaine de la nutrition animale.

On sait que la souche de *Streptomyces fradiae* peut produire au moins cinq types de protéases désignées par Ia, Ib, II, III, IV et deux peptidases (Brevet GB 1 133 579).

Le brevet FR-2034559 décrit l'utilisation de produits enzymatiques comprenant des protéases obtenues à partir de *Streptomyces fradiae,* et des compositions alimentaires animales comprenant ces produits. Mais, ce brevet ne prévoit pas un complexe enzymatique dont l'une des protéases du mélange présente un pI (point isoélectrique) de l'ordre de 7,0 et une autre présente un pI de l'ordre de 8,0. Au contraire, il n'y a pas de caractérisation d'utilisation ou de séparation des différentes protéases issues de *Streptomyces fradiae* selon leur pI.

Dans leur demande de brevet WO 87/07905, les demandeurs ont décrit un procédé d'obtention d'un complexe protéolytique composé exclusivement des protéases du type II, III et IV avec prépondérance de la protéase du type II. Ce complexe est obtenu à partir d'une souche de *Streptomyces fradiae* de type WAKSMAN 3535 par fermentation, extraction du complexe par filtration, puis ultrafiltration sur une membrane ayant un seuil de coupure correspondant à un poids moléculaire compris entre 2000 et 12 000, et enfin atomisation. Le complexe ainsi obtenu sous forme de poudre titre au moins à 2000 unités Anson/mg de protéines.

Dans cette demande, la protéase du type II a été caractérisée par un poids moléculaire voisin de 18 kDa et un point isoélectrique voisin de 9,0.

Ce complexe, utilisé dans l'alimentation animale, permet aux animaux d'assimiler efficacement les aliments riches en protéines brutes et ainsi stimule leur croissance. En outre, ce complexe, utilisé chez des truies gestantes, permet d'obtenir une augmentation du poids des portées à la naissance accompagnée d'une diminution de la consommation alimentaire et une amélioration de l'état sanitaire.

Toutefois, ce procédé ne permet pas d'éliminer efficacement des produits secondaires contaminants.

Dans la demande de brevet WO 97/37681, des améliorations sont apportées permettant l'utilisation d'un produit titrant à au moins 4 000 unités Anson/mg de protéines pour des applications thérapeutiques. Ainsi, la protéinase prépondérante présente une activité spécifique supérieure à 100 000 unités Anson/mg de protéines avec un poids moléculaire compris entre 30 et 36 kDa et un point isoélectrique voisin de 7,0.

Les analyses effectuées ultérieurement ont montré que le poids moléculaire de la protéase était de 18-20 kDa.

Les applications thérapeutiques décrites consistent essentiellement en une action positive de réduction des excès de sécrétion de mucus caractérisant certaines maladies, en une amélioration de la résorption intestinale et de la circulation sanguine ou encore en la lutte contre les maladies infectieuses. En outre, les compositions sont décrites comme facilitant la régénération des villosités atrophiées ou détruites de la muqueuse intestinale.

Au vu des propriétés thérapeutiques importantes du complexe enzymatique extrait des cultures de *Streptomyces fradiae,* il est important d'utiliser un complexe enzymatique débarrassé d'une manière plus complète des impuretés biologiques qui pourraient affecter son activité.

La présente invention vise donc à fournir une amélioration du complexe enzymatique précédemment décrit pour favoriser notamment de bonnes conditions d'élevage et la santé des animaux dans le cadre d'élevages industriels.

L'invention a donc pour objet l'utilisation d'un complexe enzymatique comprenant un mélange de protéases, obtenu par mise en culture d'une souche de *Streptomyces fradiae,* pour la supplémentation de l'alimentation des animaux d'élevage, caractérisée en ce que parmi ces protéases du mélange l'une d'elles présente un point isoélectrique de l'ordre de 7,0 et une activité spécifique d'environ 150 000 unités Anson /mg de protéines et une autre protéase présente un point isoélectrique de l'ordre de 8,0 et une activité spécifique d'environ 38 000 unités Anson/mg de protéines, ledit complexe renfermant très majoritairement ces deux types de protéases, c'est-à-dire une proportion de ces deux protéases dépassant 80% de l'activité dudit mélange, avec l'élimination de substances de charge dépourvues d'activité protéolytique dudit mélange.

Selon une caractéristique de l'invention, le complexe enzymatique est utilisé comme complément alimentaire sous forme de poudre, de liquide ou toute autre forme adaptée pour le mélange à des compositions alimentaires pour améliorer l'état général des animaux d'élevages industriels.

Selon une autre caractéristique de l'invention, le complexe enzymatique sous forme de poudre est mélangé avec une composition alimentaire à sec jusqu'à homogénéité dans un tambour rotatif.

Selon une autre caractéristique de l'invention, le complexe enzymatique sous forme liquide est pulvérisé en lit fluidisé sur une composition alimentaire, puis granulé.

Selon une caractéristique de l'invention, le complexe enzymatique est utilisé dans l'alimentation de la volaille.

Selon une autre caractéristique de l'invention, le complexe enzymatique est utilisé dans l'alimentation des porcs.

Selon une autre caractéristique de l'invention, le complexe enzymatique est utilisé dans l'alimentation des poissons.

L'invention a enfin pour objet un procédé de fabrication du complexe enzymatique selon l'invention, caractérisé en ce qu'on met en culture une souche de *Streptomyces fradiae,* en ce qu'on filtre le moût de fermentation, en ce qu'on procède ensuite à une extraction du complexe enzymatique par ultrafiltration et chromatographie échangeuse d'ions suivies d'une isoélectrofocalisation et enfin on lyophilise le complexe obtenu.

Cette invention permet de fournir un complément alimentaire dont les caractéristiques sont rigoureusement déterminées.

L'invention permet tout d'abord d'adapter les doses à administrer avec précision dans les compositions alimentaires afin d'obtenir les effets thérapeutiques souhaités.

L'invention présente également l'avantage d'améliorer l'état sanitaire des animaux dans les élevages industriels.

En outre, l'invention apporte également une amélioration de l'état général des animaux dans les élevages industriels ce qui augmente la rentabilité de l'exploitation.

L'invention permet encore une augmentation de la croissance des animaux tout en induisant une baisse de la consommation alimentaire.

D'autres caractéristiques, détails et avantages de l'invention seront mieux compris à la lecture du complément de description qui va suivre de modes de réalisation donnés à titre d'exemple en relation avec vues illustrant les villosités intestinales.

On sait que le mélange enzymatique commercialisé sous la dénomination Panstimase® (PANcreatic STIMulating diastASE) est un complexe enzymatique issu de la fermentation de *Streptomyces fradiae.* Ce complexe est décrit comme contenant majoritairement trois protéases dont certaines agissent spécifiquement sur des protéines sclérotiques (collagène, élastine, kératine), mais aussi sur certaines toxines de nature protéique libérées par le vibrion cholérique ou certaines souches pathogènes *d'Escherichia coli.*

Dans le domaine de la zootechnique, il est important de disposer d'un produit techniquement défini, parfaitement constant et dont les propriétés sont ainsi clairement déterminées.

Afin d'obtenir le complexe enzymatique selon l'invention, on met en culture une souche de *Streptomyces fradiae* et on filtre le moût de fermentation. Ensuite, on procède à une extraction et une caractérisation du complexe enzymatique par ultrafiltration, chromatographie échangeuse d'ions et isoélectrofocalisation. Enfin, on procède à une lyophilisation du complexe obtenu.

Pour la culture de la souche de *Streptomyces fradiae,* on utilise une souche par exemple de type WAKSMAN 3535 et pouvant être génétiquement améliorée pour obtenir notamment une augmentation de l'activité protéolytique ou la suppression de toute sécrétion d'antibiotiques.

La culture se déroule en fermenteur de production industrielle dans un milieu de culture adapté. Ce milieu de culture peut par exemple être à base de farine de soja: 15 g/l; glucose: 30 g/l ; phosphate bipotassique: 1 g/l; carbonate de calcium: 10 g/l.

La réaction se déroule à un pH compris entre 7,0 et 7,5, à une température de fermentation d'environ 28°C avec une aération à 0,3 volume d'air stérile par volume de milieu et par minute

Une fois la culture terminée, le mycélium est éliminé par filtration du moût de fermentation en présence d'un agent clarifiant.

L'extraction du complexe enzymatique nécessite la mise en oeuvre de plusieurs techniques dont l'ultrafiltration, la chromatographie échangeuse d'ions et l'isoélectrofocalisation.

### Ultrafiltration

L'ultrafiltration du filtrat est réalisée à l'aide d'une membrane ayant un seuil de coupure de 50kDa.

Après 3h de filtration, le filtrat recueilli (71% du volume de départ) présente une activité protéolytique révélée par le test au film de gélatine. Ce test consiste en la dégradation d'un film de gélatine coloré à l'argent et déposé sur un support solide.

### Chromatographie échangeuse d'ions

On utilise une chromatographie échangeuse de cations réalisée avec des colonnes de carboxyméthyl gel Cellufine C-500 (Amicon) de dimensions adaptées.

Le tampon de chromatographie est un tampon citrate 10 mM à pH 5,5 et l'élution est réalisée par un gradient de NaCl de 0 à 1,0 M. Le débit d'élution est adapté en fonction de la taille des colonnes, par exemple il est de 10 à 15 ml/min pour une colonne de 2,5 cm de diamètre et de 27 cm de hauteur.

### Isoélectrofocalisation

L'isoélectrofocalisation analytique est réalisée avec des gels prêts à l'emploi de la société Pharmacia pour des pH allant de 3 à 9 sur Phast-system. La révélation se fait au bleu de Coomassie.

Dans le cas de l'isoélectrofocalisation préparative en milieu solide, la solution enzymatique est dialysée contre de l'eau distillée (95 ml) et mélangée à 5 ml d'ampholytes 3-9 avec 4,7 g de gel Ultrodex et 0,5 g de glycine. Le gel est asséché pendant 6h à 40°C. La migration a lieu pendant la nuit, ou période équivalente, à puissance constante de 3W. La révélation se fait au bleu de Coomassie.

Les différentes bandes de gel sont prélevées, rincées avec 3 ml d'eau, filtrées, puis rincées avec 3 ml de tampon Tris-HCl.

Les fractions ainsi obtenues sont ensuite analysées pour leur contenu en activité protéolytique et leur teneur en protéines. Les échantillons sont ensuite dialysés contre le tampon Tris-HCl 0,3 M pH 8,0.

Dans le cas de l'isoélectrofocalisation préparative en milieu liquide, la solution enzymatique dialysée contre de l'eau distillée est mélangée aux ampholytes (3,5 ml). La migration se fait à puissance constante 8 W pendant 4H.

Les fractions prélevées sont analysées puis dialysées contre du tampon Tris-HCl 0,3 M pH 8.0.

Les conditions expérimentales indiquées ci-dessus sont à adapter selon les quantités de produits que l'on souhaite obtenir et l'utilisation en conditions industrielles.

On obtient ainsi dans le mélange une protéase majoritaire ayant un point isoélectrique (pI) de l'ordre de 7,0 et dans une moindre proportion une protéase de pI de l'ordre de 8,0.

La protéase majoritaire de pI 7,0 environ est obtenue dans un volume de 9 ml avec une concentration protéique de 0,49 mg/ml. Des précautions particulières de purification peuvent être nécessaires du fait de la précipitation de cette protéase à son point isoélectrique. La solution obtenue a un titre de l'ordre de 75 000 U.A/ml.

Cette protéase est purifiée après homogénéisation à l'échelle de 4 mg avec une activité spécifique de 150 000 U.A/mg de protéines.

Une unité Anson (U.A.) est ici définie comme étant la quantité d'enzyme qui, incubée pendant 10 minutes à 25°C et à pH 7,5 en présence d'hémoglobine dénaturée, libère de ce substrat l'équivalent de 1 microgramme de tyrosine, déterminée par absorption spectrophotométrique à 280 nm sur le filtrat non précipitable à l'acide trichloracétique.

La protéase de pI 8,0 environ est récupérée conjointement avec la précédente mais dans un volume plus important, de 35 ml par exemple, et présente une concentration en protéine de 1,48 mg/ml. Le titre de cette solution est de l'ordre de 56000 U.A/ml.

Cette protéase est purifiée après homogénéisation à l'échelle de 50 mg avec une activité spécifique de l'ordre de 38 000 U.A/mg de protéines.

Le complexe enzymatique purifié précédemment décrit contenait essentiellement des protéases et d'autres protéines. Les purifications entreprises et la technique d'électrophorèse utilisée apporte la preuve d'une purification supplémentaire et de l'élimination de substances de charge dépourvues d'activité protéolytique.

Les différentes protéases produites peuvent être mieux caractérisées en déterminant leurs activités sur des protéines telles que le collagène, la kératine, l'élastine ou l'hémoglobine.

Ces déterminations sont malheureusement complexes en raison de leur manque de sensibilité et de leur manque de spécificité. Il est donc nécessaire de recourir à plusieurs tests d'activité, constituant des méthodes de détection, et principalement la méthode Anson.

### Tests d'activités :

### Test d'Anson :

Ce test permet de doser une activité protéolytique, sans spécificité. Il consiste à doser l'hydrolyse de l'hémoglobine dénaturée par détermination spectrophotométrique de la tyrosine contenue dans les oligopeptides libérés.

### Test à l'azocaséine :

Ce test consiste à déterminer par spectrophotométrie à 440 nm, l'hydrolyse de la caséine sur laquelle a été fixé un colorant orange. Ce test n'est pas spécifique. Après incubation avec l'enzyme, l'azocaséine restante est précipitée à l'acide trichloroacétique. La mesure de la densité optique reflète la présence des peptides libérés, donc l'activité enzymatique.

### Test d'activité élastolytique :

### Test à l'élastine Congo-Red

Ce test consiste à doser spectrophotométriquement à 495 nm, les peptides colorés libérés par l'hydrolyse de l'élastine sur laquelle a été fixé un colorant rouge.

Le problème éventuel de ce dosage vient de l'insolubilité du substrat et donc sa sensibilité dépend de la précision sur le prélèvement de celui-ci, de l'agitation du milieu d'incubation et de l'adhérence du substrat sur les parois du récipient d'incubation.

Les essais réalisés montrent un manque de linéarité entre l'activité et la quantité d'enzyme. On observe bien une linéarité entre l'activité et le temps d'incubation, mais la droite le représentant ne passe pas par l'origine.

On peut donc mesurer une différence d'absorbance entre deux temps d'incubation (20 et 40 min). Avec 10 µl de complexe dilué 10 fois, on mesure une variation de 0,049µDO/min. Le complexe enzymatique de l'invention présente donc bien une activité protéolytique vis-à-vis de l'élastine.

### Test au NBA

### (N-Boc-L-Alaninate-para-nitrophénylester)

Ce test consiste à doser spectrophotométriquement à 347,5 nm, l'activité estérase présente dans l'élastase par la détermination du para-nitrophénol libéré *(*Visser L.et al.Biochim, Biophys Acta 268 (1972) 207.260*).*

Ce dosage n'est pas spécifique d'une activité élastolytique, mais il est très rapide (cinétique de 3 min) et présente une bonne sensibilité.

En effet, avec 50 µl de complexe dilué 100 fois, on mesure une variation de 0,235 µDO/min. Le complexe enzymatique présente donc une activité permettant la libération de para-nitrophénol.

### Test d'activité collagènolytique :

### Test à l'azocoll

Ce test consiste à doser spectrophotométriquement à 520 nm, les peptides libérés par l'hydrolyse de l'azocoll (collagène broyé et sur lequel est fixé un colorant rouge-bordeaux) *(*Chavira, Analytical Biochemistry 136 (1984) 446-450*).*

Ce test présente les mêmes inconvénients que celui de l'élastine Congo-Red (substrat insoluble), mais présente une bonne sensibilité : avec 50 µl de complexe dilué 50 fois, on mesure une DO de 0,398 au bout de 10 min d'incubation. Le complexe enzymatique de l'invention présente donc une activité protéolytique vis-à-vis de l'azocoll.

On constate ainsi l'activité protéolytique du complexe enzymatique selon l'invention. La purification dudit complexe n'a pas altéré son activité.

Le complexe protéique ainsi obtenu et caractérisé trouve un emploi en thérapeutique animale, notamment comme agent immunostimulant et comme agent de régénération des Plaques de Peyer, en particulier chez les sujets âgés.

En outre, le complexe enzymatique permet de régénérer les villosités intestinales chez les animaux âgés (rats, poulets, etc) et donc améliore grandement l'absorption intestinale des nutriments d'intérêt.

Le complexe enzymatique selon la présente invention est destiné principalement à la zootechnie et plus particulièrement à l'amélioration de l'alimentation du bétail (bovins, porcins, ovins), de la volaille, des lapins et des poissons et tout autre animal monogastrique.

Incorporé à des doses allant de 0,025 à 1 g/kg à des compositions alimentaires, ce complexe enzymatique améliore le métabolisme de ces animaux et contribue à leur assurer un meilleur état sanitaire. De préférence, les doses de complexe introduites dans les compositions alimentaires sont de 0,05 à 0,2 g/kg.

Dans le cas de l'élevage en batterie, le maintien de l'état sanitaire joue un rôle important car il évite la propagation de maladies dues à la promiscuité ou au confinement. On diminue ainsi sensiblement la morbidité surtout la mortalité des animaux d'élevage par contamination bactérienne.

Les compositions alimentaires à base de ce mélange enzymatique contiennent un ou plusieurs excipients ou véhicules à visée nutritive comme par exemple des farines de céréales (blé, froment, maïs, seigle, riz, mil, sorgho), de soja, des glucides (lactose, mannose), des éléments de charge (caséine, son, cellulose, dérivés de la cellulose) et/ou des éléments minéraux (craie, argile, bentonite, silice) et tout autre élément utilisé en nutrition animale.

Ces compositions sont mélangées avec le complexe à sec jusqu'à homogénéité dans un tambour rotatif ou bien le complexe enzymatique, sous forme liquide et en particulier aqueuse, est pulvérisé en lit fluidisé sur une composition alimentaire, puis granulé. De telles opérations peuvent être conduites à des températures variables allant de 15°C à 60°C.

L'utilisation d'un complexe enzymatique plus pur et plus actif permet ainsi de doser plus précisément l'activité biologique des compositions nutritives selon l'invention et d'assurer une homogénéité satisfaisante des résultats obtenus.

De ce fait, les compositions alimentaires selon l'invention ont une teneur en complexe enzymatique clairement déterminée et bien constante.

Ainsi, voici quelques exemples non limitatifs de compositions alimentaires destinées aux animaux d'élevages contenant le complexe enzymatique selon l'invention en mélange avec des excipients ou véhicules appropriés à visé nutritive.

### Composition alimentaire pour volaille :

On prépare une composition pour un lot de 10 kg en ajoutant le complexe enzymatique :

| | |
|---|---|
| Farine de blé | 2,5 kg |
| Lactose | 4,5 kg |
| Son | 3 kg |
| Complexe enzymatique de l'invention | 0,2 g/kg |

Une telle composition est incorporée à raison de 0,5 kg par tonne de nourriture pour la volaille.

### Composition alimentaire pour volaille :

On prépare une composition pour un lot de 20,1 kg à laquelle on ajoute le complexe enzymatique que l'on incorpore dans la nourriture de la volaille.

| | |
|---|---|
| Farine de blé | 1,5 kg |
| Farine d'avoine | 4,5 kg |
| Cellulose | 14 kg |
| Silice colloïdale | 0,100 kg |
| Complexe enzymatique de l'invention | 0,1 g/kg |

### Composition alimentaire porcins :

On prépare une composition pour un lot de 10 kg à laquelle on ajoute le complexe enzymatique que l'on incorpore dans la nourriture des porcs.

| | |
|---|---|
| Farine de blé | 4 kg |
| Son | 2 kg |
| Caséine | 3 kg |
| Fécule de pomme de terre | 1 kg |
| Complexe enzymatique de l'invention | 0,2 g/kg |

### Composition alimentaire bétail :

On prépare de la même manière la composition suivante :

| | |
|---|---|
| Amidon de riz | 0,5 kg |
| Fécule de pomme de terre | 2,5 kg |
| Complexe enzymatique de l'invention | 0,2 g/kg |

Le premix ainsi constitué est additionné en petites fractions à un mélange de 6 kg de poudre de protéines de mouton et de 14 kg de caséine. La préparation homogénéisée est destinée à être incorporée dans la nourriture du bétail à raison de 0,5 kg par tonne d'aliment.

### Composition alimentaire pour volaille :

On prépare la composition pour un lot de 10 kg à laquelle on ajoute le complexe enzymatique :

| | |
|---|---|
| Maïs | 5,65 kg |
| Farine de soja | 3,23 kg |
| Farine de graine de coton | 0,3 kg |
| Farine de germe de blé | 0,4 kg |
| Calcaire | 0,13 kg |
| Phosphate di calcique | 0,16 kg |
| NaCl | 0,03 kg |
| Complexe aminoacide | 0,1 kg |
| Complexe enzymatique de l'invention | 0,2 g/kg |

Le complexe enzymatique selon l'invention peut également être ajouté à des compositions alimentaires du commerce pour volaille afin de supplémenter ces préparations.

### Composition alimentaire pour les poissons :

On prépare la composition suivante que l'on incorpore dans l'alimentation pour poissons selon les pratiques habituelles.

| | |
|---|---|
| Farine de poisson | 50,8 % |
| Graisses et huiles animales | 28 % |
| Farine de soja | 9,9 % |
| Farine de blé | 10 % |
| Complexe vitaminique (A, D, E, C...) | 1 % |
| Complexe enzymatique de l'invention | 0,3 % |

### Partie expérimentale :

Pour déterminer l'action du complexe de l'invention sur les villosités intestinales, on réalise des essais sur des rats relativement âgés (plus de sept mois).

Ces rats reçoivent une alimentation à base de protéines exclusivement végétales du type farine de blé, de soja, de maïs. On ajoute dans cette alimentation le complexe enzymatique selon l'invention comme décrit précédemment à raison de 0,1 ou 0,2 g/kg.

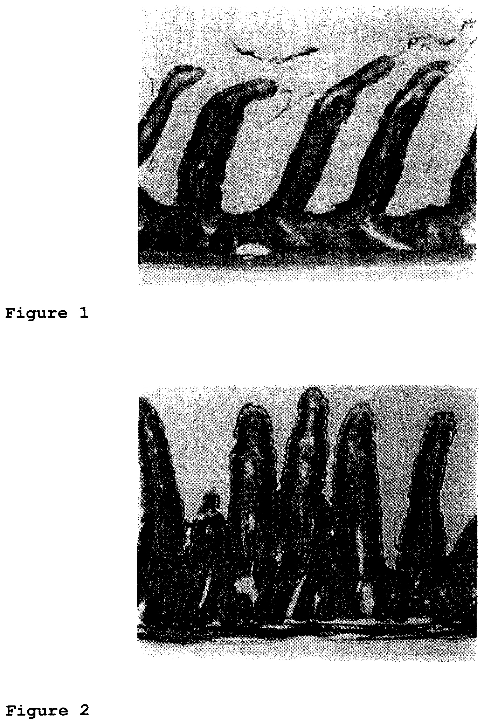

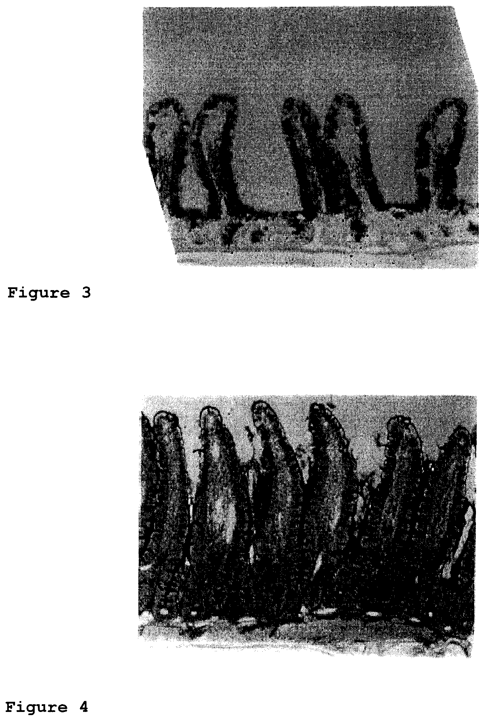

Les résultats sont explicités sur les figures 1 à 4. Ces figures sont des vues en microscopie photonique, (grossissement x120), des villosités du duodénum et du jéjunum.

La figure 1 représente les villosités du duodénum des rats n'ayant pas reçu dans leur alimentation le complexe enzymatique selon l'invention.

La figure 2 représente les villosités du duodénum des rats ayant reçu l'alimentation incorporant le complexe enzymatique selon l'invention.

La figure 3 montre les villosités du jéjunum des rats n'ayant pas reçu dans leur alimentation le complexe enzymatique selon l'invention.

La figure 4 montre les villosités du jéjunum des rats ayant reçu l'alimentation incorporant le complexe enzymatique selon l'invention.

On constate ainsi que l'addition du complexe enzymatique dans l'alimentation des animaux âgés (rats, poulets, etc) augmente la taille et le nombre de villosités dans le duodénum comme dans le jéjunum. On a trouvé des augmentations allant jusqu'à 70%.

Ces résultats sont transposables pour les microvillosités intestinales. On obtient également des résultats similaires chez les poulets, les porcs et les poissons.

L'augmentation de surface développée permet une absorption plus rapide des nutriments ou encore des médicaments.

Cette amélioration de l'absorption est observée chez des truites recevant une alimentation contenant un antibiotique. Dans le test, l'alimentation contient 4g/kg d'oxytétracycline et les résultats sont lus après 48 heures. On retrouve alors une augmentation de la concentration tissulaire en antibiotique de l'ordre de 100% dans le foie (5,75 au lieu de 2,5 microgramme/g), 400% dans les muscles (1,75 au lieu de 0,32 microgramme/g) et 1000% dans les reins (5,75 au lieu de 0,5 microgramme/g).

On obtient des résultats similaires chez les poulets et chez les porcs.

Cette augmentation de la densité peut également être à l'origine d'une augmentation de la production locale en composés physiologiquement actifs tels que les hormones intestinales.

## Revendications

1. Utilisation d'un complexe enzymatique comprenant un mélange de protéases, obtenu par mise en culture d'une souche de *Streptomyces fradiae,* pour la supplémentation de l'alimentation des animaux d'élevage, **caractérisée en ce que** parmi ces protéases du mélange l'une d'elles présente un point isoélectrique de 7,0 et une activité spécifique de 150 000 unités Anson /mg de protéines et une autre protéase présente un point isoélectrique de 8,0 et une activité spécifique de 38 000 unités Anson/mg de protéines, ledit complexe renfermant très majoritairement ces deux types de protéases, c'est-à-dire une proportion de ces deux protéases dépassant 80% de l'activité dudit mélange, avec l'élimination de substances de charge dépourvues d'activité protéolytique dudit mélange.

2. Utilisation selon la revendication 1 du complexe enzymatique comme complément alimentaire sous forme de poudre, de liquide ou toute autre forme adaptée pour le mélange à des compositions alimentaires pour améliorer l'état général des animaux d'élevage industriel.

3. Utilisation selon la revendication 2 dans laquelle le complexe enzymatique sous forme de poudre est mélangé avec une composition alimentaire à sec jusqu'à homogénéité dans un tambour rotatif.

4. Utilisation selon la revendication 2 dans laquelle le complexe enzymatique sous forme liquide est pulvérisé en lit fluidisé sur une composition alimentaire, puis granulé.

5. Utilisation selon l'une des revendications précédentes dans l'alimentation de la volaille.

6. Utilisation selon l'une des revendications 1 à 4 dans l'alimentation des porcs.

7. Utilisation selon l'une des revendications 1 à 4 dans l'alimentation des poissons.

8. Procédé de fabrication du complexe enzymatique selon l'une des revendications 1 à 4, **caractérisé en ce qu'**on met en culture une souche de *Streptomyces fradiae,* **en ce qu'**on filtre le moût de fermentation, **en ce qu'**on procède ensuite à une extraction du complexe enzymatique par ultrafiltration et chromatographie échangeuse d'ions suivies d'une isoélectrofocalisation et enfin on lyophilise le complexe obtenu.

## Patentansprüche

1. Verwendung eines Enzymkomplexes, welcher ein Gemisch von Proteasen aufweist, welche durch Kultivieren eines Stamms von *Streptomyces fradiae* zur Ergänzung der Nahrung von Nutztieren erzielt wird, **dadurch gekennzeichnet, dass** unter diesen Proteasen des Gemisches eine von ihnen einen isoelektrischen Punkt von 7,0 und eine spezifische Aktivität von 150000 Anson-Einheiten/mg Protein aufweist, und eine andere Protease einen isoelektrischen Punkt von 8,0 und eine spezifische Aktivität von 38000 Anson-Einheiten/mg Protein aufweist, wobei der genannte Komplex mit überwiegender Mehrheit diese beiden Arten von Proteasen enthält, das heißt, wobei ein Anteil dieser beiden Proteasen 80% der Aktivität des genannten Gemisches übersteigt bei der Beseitigung von Füllstoffen ohne proteolytische Aktivität des genannten Gemisches.

2. Verwendung nach Anspruch 1 des Enzymkomplexes als Nahrungsergänzungsmittel in Form von Pulver, Flüssigkeit oder jeder anderen Form, welche für die Vermischung mit Nahrungszusammensetzungen geeignet ist, um den Allgemeinzustand der industriellen Nutztiere zu verbessern.

3. Verwendung nach Anspruch 2, bei welcher der Enzymkomplex in Form von Pulver mit einer trockenen Nahrungszusammensetzung bis zur Homogenität in einer rotierenden Trommel vermischt wird.

4. Verwendung nach Anspruch 2, bei welcher der Enzymkomplex in flüssiger Form in einer Wirbelschicht auf eine Nahrungszusammensetzung gesprüht und dann granuliert wird.

5. Verwendung nach einem der vorhergehenden Ansprüche bei der Geflügelnahrung.

6. Verwendung nach einem der Ansprüche 1 bis 4 bei der Schweinenahrung.

7. Verwendung nach einem der Ansprüche 1 bis 4 bei der Fischnahrung.

8. Verfahren zur Herstellung des Enzymkomplexes nach einem der Ansprüche 1 bis 4, **dadurch gekennzeichnet, dass** ein Stamm von *Streptomyces Fradiae* kultiviert wird, dass die Gärmaische gefiltert wird, dass dann mit einer Extraktion des Enzymkomplexes durch Ultrafiltration und Ionenaustauschchromatographie fortgefahren wird, was von einer Isoelektrofokussierung gefolgt wird, und schließlich der erhaltene Komplex lyophilisiert wird.

## Claims

1. Utilization of an enzymatic complex comprising a mixture of proteases obtained by culturing a *Streptomyces fradiae* strain to supplement farm animals feed, **characterized in that** among this mixture of proteases one of them has an isoelectric point of 7.0 and a specific activity of 150,000 Anson units/mg of protein and another proteases has an isoelectric point of 8.0 and a specific activity of around 38,000 Anson units/mg of protein, the said complex predominantly contains these two types of proteases, in other words a proportion of these two proteases exceeding 80% of the activity of said mixture, and with elimination of contaminating materials that have no proteolytic activity from said mixture.

2. Utilization according to Claim 1 of the enzymatic complex as a feed supplement in powder, liquid or any other form suited to its mixture with feed compositions to improve the general condition of factory farm animals.

3. Utilization according to Claim 2, in which the enzymatic complex in powder form is dry mixed with a feed composition in a rotating drum until homogenized.

4. Utilization according to Claim 2, in which the enzymatic complex in liquid form is pulverized in a fluidized bed onto a feed composition, and is then granulated.

5. Utilization according to one of the above Claims in poultry feed.

6. Utilization according to one of the above Claims 1 to 4 in pig feed.

7. Utilization according to one of the above Claims 1 to 4 in fish feed.

8. Manufacturing process for the enzymatic complex according to one of the above Claims 1 to 4, **characterized in that** a *Streptomyces fradiae* strain is cultured, **in that** the fermentation broth is filtered, **in that** the enzymatic complex is thereafter extracted by ultrafiltration and ion exchange chromatography followed by an isoelectric focusing and finally, the complex thus obtained is lyophilized.
